# EUROPEAN PATENT SPECIFICATION

(11) **EP 4 326 909 B1**
(45) Date of publication and mention of the grant of the patent: **21.01.2026**
(21) Application number: 22839992.9
(22) Date of filing: 30.11.2022
(51) Int. Cl.: C12Q 1/70

(54) **COMPOSITION, KIT, METHOD FOR DETECTING HIV-1 AND USE THEREOF**
ZUSAMMENSETZUNG, KIT, VERFAHREN ZUM NACHWEIS VON HIV-1 UND VERWENDUNG DAVON
COMPOSITION, KIT, PROCÉDÉ DE DÉTECTION DU VIH-1 ET SON UTILISATION

(30) Priority: 27.01.2022 CN 202210102254
(43) Date of publication of application: 28.02.2024
(73) Proprietor: Sansure Biotech Inc., Changsha, Hunan 410205 (CN)
(72) Inventor: DAI, Lizhong, Changsha, Hunan 410205 (CN); DING, Feng, Changsha, Hunan 410205 (CN); LIU, Xiaoming, Changsha, Hunan 410205 (CN); REN, Xiaomei, Changsha, Hunan 410205 (CN); LIU, Jia, Changsha, Hunan 410205 (CN); DENG, Zhongping, Changsha, Hunan 410205 (CN)
(74) Representative: RGTH
(86) International application number: PCT/CN2022/135446
(87) International publication number: WO 2023/142656

(56) References cited:
- WO-A1-2016/137975
- CN-A- 107 090 521
- TANG ET AL: "A RealTime HIV-1 viral load assay for automated quantitation of HIV-1 RNA in genetically diverse group M subtypes A-H, group O and group N samples", JOURNAL OF VIROLOGICAL METHODS, ELSEVIER BV, NL, vol. 146, no. 1-2, 3 November 2007 (2007-11-03), pages 236 - 245, XP022327160, ISSN: 0166-0934, DOI: 10.1016/J.JVIROMET.2007.07.003
- VENER TANYA ET AL: "Use of multiple competitors for quantification of human immunodeficiency virus type 1 RNA in plasma", JOURNAL OF CLINICAL MICROBIOLOGY, AMERICAN SOCIETY FOR MICROBIOLOGY, US, vol. 36, no. 7, 1 July 1998 (1998-07-01), pages 1864 - 1870, XP002546533, ISSN: 0095-1137

## Description

### RELATED APPLICATIONS

The present application claims priority to China Patent Application No. 202210102254.9, filed on January 27, 2022.

### TECHNICAL FIELD

The present application belongs to the field of molecular biological detection. Particularly, the present application relates to the detection of HIV-1.

### BACKGROUND

Acquired immune deficiency syndrome (AIDS) is caused by the human immunodeficiency virus (HIV). AIDS is one of the important public health problems affecting public health. HIV is a highly mutated virus with each gene having different degrees of mutation. Env gene has the highest mutation rate. The main reasons for HIV mutation comprise random mutagenesis caused by the reverse transcriptase having no proofreading ability; virus replication in vivo at high frequency; immune selective pressure of the host; genetic recombination between viral DNA and host DNA; and drug selective pressure. Non-standard ART and poor patient compliance are main cause for drug resistance variation.

Laboratory tests for HIV/AIDS patients mainly comprise HIV antibody tests, HIV nucleic acid qualitative and quantitative tests, CD4⁺ T lymphocyte count, HIV drug resistance tests, etc. The HIV-1/2 antibody tests are the gold standard for the diagnosis of HIV infection, and the HIV nucleic acid tests (both qualitative and quantitative) are also used for the diagnosis of HIV infection. The HIV antibody tests comprise screening tests and supplementary tests. The HIV supplementary tests comprise supplementary antibody tests (confirmation tests for antibodies) and supplementary nucleic acid tests (qualitative and quantitative nucleic acid tests). HIV nucleic acid quantification and CD4⁺ T lymphocyte count are two important indicators for assessment of disease progression, clinical medication, efficacy and prognosis. HIV drug resistance tests can guide the selection and replacement of ART regimens.

It is difficult to amplify all genotypes by conventional fluorescence quantitative PCR detection with only one set of primers and probes. Only several main types such as A, B, C, BC, AE can be detected. As a result, false negative or inaccurate quantification for respective subtypes may occur, and especially, group O viruses are more prone to false negative. The sensitivity of the current HIV-1 RNA fluorescence quantitative PCR reagents is greater than or equal to 50IU/ml. Viral loads at low concentrations cannot be accurately detected and quantified. A same kind of reagent with excellent performance is the fully enclosed diagnostic system produced by Roche and called "Roche Cobas6800/8800 TaqMan HIV-1 assay". The HIV-RNA in clinical sample can be quantified by extracting RNA using the principle of magnetic bead method in the fully automated instrument, then automatically adding sample and carrying out fluorescence PCR amplification of RNA. The diagnostic system has the advantages of high automation, simple operation and high sensitivity for detection (greater than 22IU/ml), but may brought great financial pressure to the construction of medical and health system in China due to the closed system and the high cost of the reagents and consumables. Each of CN107090521, WO2016137975 and Tang et al (J. Virol. Methods, vol. 146, pp.236-245, 2000) discloses RT-PCR for detection of different HIV-1 subtypes.

There is a need in the art for a HIV-1 detection reagent which enables more accurate detection of HIV-1 with high sensitivity and low cost and without false negative.

### SUMMARY

Accordingly, in a first aspect, the present application provides a composition for detecting HIV-1, comprising:
a forward primer HIV-ZF1: 5'-CCTGGGAGCTCTCTGGCTA-3' (SEQ ID NO: 1);
a reverse primer HIV-ZR1: 5'-AAGCACTCAAGGCAAGCTTTAT-3' (SEQ ID NO: 2);
a detection probe HIV-ZP1: 5'-AGGCTTAAGCAGTGGGTTCC-3' (SEQ ID NO: 3);
a forward primer Z-POL-F1: 5'-AAATCACTCTTTGGCARCGAC-3' (SEQ ID NO: 4);
a reverse primer Z-POL-R1: 5'-CCCCCTATCATTTTTGGTTTC-3' (SEQ ID NO:5);
a forward primer Z-POL-FO: 5'-ATCCCTCTTTGGGACAGACC-3' (SEQ ID NO:6); and
a detection probe Z-POL-P1: 5'-ACTGTATCATCTGCYCCTGT-3' (SEQ ID NO:7).

HIV-1 can be detected using the composition of the present application with a sensitivity of 22 IU/ml. Moreover, two sets of primers and probes for two different regions and an additional forward primer are combined in the composition of the present application to prepare a reaction system, and the HIV-1 RNA are detected using the reaction system to identify a positive reaction, effectively reducing the probability of false negative, especially, of group O viruses. There is no need for a fully enclosed diagnostic system, which effectively reduces costs.

Further, the above detection probes have fluorescent groups which are different from each other and non-interfering with each other.

Further, the above detection probes have fluorescent groups which may be the same.

Herein, "different from each other and non-interfering with each other" means that the fluorescent groups used in each set of the probes in the composition are different and will not affect the detection using each other, that is, their detection can be performed in different channels. For example, FAM, HEX, ROX and CY5 do not have close absorbance values and thus different channels can be selected without interference with each other

Further, the composition includes an internal standard forward primer, an internal standard reverse primer, and an internal standard probe, for monitoring.

Further, the internal standard includes an exogenous non-human genomic internal standard.

In a specific embodiment, the internal standard sequence (partial sequence of papillomavirus from the family Papovaviridae) is:

5’-
TTGTAAGTGTGAAGCCAGAATTGAGCTAGTAGTAGAAAGCTCAGCAGACGACC
TTCGAGCATTCCAGCAGCTGTTTCTGAACACCCTGTCCTTTGTGTGTCCGTGGT
GAAGAGTTTATTAATGAAGATATTGTAAATTTGCCCGAGAATGTCGATTGGAGA
AAAAAAGGAGCTGTCACTCCCGTAAAACATCAGGGTTCATGCGGTAGTTGTTG
GGCATTCTCGGCCGTTGCAACTGTAGAGGGAATAAATAAGATTAGAACTGGAAA
ATTAGTAGAATTATCAGAGCAAGAACTTGTTGACTGTGAAAGACGTAGCCATGG
GGCAAAGACCGTTGTGTCCAGAAGAAAAACAAAGACATTTGGATAAAAAGAA
ACGATTCCACAACATAGGAGGAAGGTGGACAGGACGTTGCATAGCATGTTGGA
GAAGACCTCGTACTGAAACCCAAGTGTAAACATGCGATATTTTATACT-3 (SEQ ID
NO:11).

In a specific embodiment, the composition further includes: an exogenous non-human genomic internal standard forward primer as set forth in HPGT31-F: 5'-CAAAGACCGTTGTGTCCAGAAG-3' (SEQ ID NO:8), an exogenous non-human genomic internal standard reverse primer as set forth in HPGT31-R: 5'-CGCATGTTTACACTTGGGTTTC-3' (SEQ ID NO:9), and an exogenous non-human genomic internal standard probe as set forth in HPGT31-P: 5'-CAACGTCCTGTCCACCTTCCTCCTATG-3' (SEQ ID NO:10).

Further, the internal standard probe and the detection probe have fluorescent groups which are different from each other and non-interfering with each other.

In the present application, the fluorescent reporter groups may be selected from, but is not limited to, FAM, HEX, ROX, VIC, CY5, 5-TAMRA, TET, CY3 and JOE.

In a specific embodiment, the fluorescent groups of the detection probes are FAM.

In a specific embodiment, the fluorescent groups of the detection probes are FAM and CY5.

In a specific embodiment, the fluorescent group of the internal standard probe is HEX.

Furthermore, the probe further has a quenching group, such as BHQ1 or BHQ2, at the 3' end.

In a specific embodiment, the probe has BHQ1 at the 3' end.

Further, the primers are present in the composition in an amount of 0.1-0.3 µM; and the probes are present in the composition in an amount of 0.05-0.20 µM.

In a specific embodiment, the components in the compositions of the present application are present in a mixture.

In a second aspect, the present application provides use of the above-mentioned composition of the present application in preparing a kit for detecting HIV-1.

In a third aspect, the present application provides a kit for detecting HIV-1, comprising the above-mentioned composition of the present application or the nucleic acid components of the above-mentioned composition of the present invention wherein said components are present in separate packages.

Furthermore, the kit further includes a negative quality control and a positive quality control.

In a specific embodiment, the negative quality control is at least one of DEPC H2O, normal saline, TE buffer, inactivated human immunodeficiency virus-negative plasma, and the positive quality control is a pseudovirus containing the internal standard gene sequence, serially diluted in 0.01% TE-SDS diluent.

Further, the kit further includes a nucleic acid release system and a nucleic acid amplification system.

Further, the nucleic acid release system includes an RNA extraction solution containing magnetic beads.

Further, a washing liquid 2 in the RNA extraction solution contains silicone oil.

In a specific embodiment, the silicone oil plays the role in protecting the PCR reaction solution. Reagent evaporation and aerosol contamination can be effectively reduced during heating and amplification at the high temperature.

Further, the kit includes 4 standard products (i.e. quantitative reference) at different amounts. The standard products are a pseudovirus containing a known gene segment RNA.

An unknown sample and the standard products are subjected to reaction in the same fluorescent PCR experiment. Ct values obtained from the standard products are fitted to a standard curve to calculate the amount of the unknown sample. The negative and positive controls are provided for the operation and quantitative calculation of the kit for quality control.

Further, the kit includes at least one of dNTPs, a PCR buffer, Mn²⁺ and Mg²⁺.

Further, the kit includes at least one of a nucleic acid release agent, a nucleic acid extraction reagent, reverse transcriptase, uracil glycosylase and DNA polymerase.

Further, the kit includes at least one of a nucleic acid release reagent, a nucleic acid extraction reagent, dNTPs, reverse transcriptase, uracil glycosylase, DNA polymerase, a PCR buffer and Mg²⁺.

Further, Mn²⁺ is manganese acetate at 10-1000 mM.

Further, the reverse transcriptase is at a concentration of 5 U to 15 U per reaction, and for example, the reverse transcriptase can be murine leukemia reverse transcriptase (MMLV) or Tth enzyme; the DNA polymerase is at a concentration of 3 U to 15 U per reaction, and for example, the DNA polymerase can be Taq enzyme.

In a specific embodiment, the kit of the present application includes reverse transcriptase, Taq enzyme, uracil glycosylase, Mg²⁺, Mn²⁺, Rnasin, dNTPs, primers, probes and a PCR buffer.

Common PCR buffers are composed of buffer systems such as Tris-HCl, MgCl₂, KCl, and Triton X-100. Generally, the total volume in a single PCR reaction tube is 20 µl to 100 µl.

In a specific embodiment, the kit of the present application is compatible with a digital PCR amplification system, that is, the kit can be directly used for amplification on a digital PCR instrument.

In a fourth aspect, the present application provides a method for detecting HIV-1, comprising:
1) extracting or releasing nucleic acids from a sample to be tested;
2) subjecting the nucleic acids obtained in 1) to fluorescence quantitative PCR using the above-mentioned composition of the present application or the above-mentioned kit of the present application; and
3) obtaining a result and analyzing the result.

In the present application, the sample to be tested may be semen, vaginal discharge, tissue fluid, blood, etc., but is not limited thereto.

Further, the fluorescence quantitative PCR is performed under the following conditions:

1 cycle of reverse transcription at a temperature of 50-60°C for 5-30 min; 1 cycle of cDNA pre-denaturation at a temperature of 95°C for 1-10 min; and 40-50 cycles of denaturation at a temperature of 95°C for 5-20 sec and annealing at 55-60°C for 20-60 sec, with fluorescence collecting.

In a specific embodiment, the fluorescence quantitative PCR is performed under the following conditions: 1 cycle of reverse transcription at a temperature of 60°C for 30 min; 1 cycle of cDNA pre-denaturation at a temperature of 95°C for 1 min; and 45 cycles of denaturation at a temperature of 95°C for 15 sec and annealing at 58°C for 10 sec, with fluorescence collecting.

In a specific embodiment, there is provided a method for detecting HIV-1, comprising:
1) extracting or releasing nucleic acids from a sample to be tested;
2) subjecting the nucleic acids obtained in 1) to fluorescence quantitative PCR using the above-mentioned composition of the present application or the above-mentioned kit of the present application, and
3) obtaining a result and analyzing the result.

Further, the fluorescence quantitative PCR is performed under the following conditions:

1 cycle of reverse transcription at a temperature of 50-60°C for 5-30 min; 1 cycle of cDNA pre-denaturation at a temperature of 95°C for 1-10 min; and 40-50 cycles of denaturation at a temperature of 95°C for 5-20 sec and annealing at 55-60°C for 20-60 sec, with fluorescence collecting.

In a specific embodiment, the fluorescence quantitative PCR is performed under the following conditions: 1 cycle of reverse transcription at a temperature of 60°C for 30 min; 1 cycle of cDNA pre-denaturation at a temperature of 95°C for 1 min; and 45 cycles of denaturation at a temperature of 95°C for 15 sec and annealing at 58°C for 10 sec, with fluorescence collecting.

### BRIEF DESCRIPTION OF DRAWINGS

FIGS. 1-2 shows the detection sensitivity of the composition of the present application;
FIG. 3 shows the detection of clinical samples using a single set of primers and probe, HIV-ZF/R/P;
FIG. 4 shows the detection of clinical samples using a single set of primers and probe, Z-POL-F/R/P;
FIG. 5 shows the detection of clinical samples using two sets of primers and probes, HIV-ZF/R/P and Z-POL-F/R/P;
FIG. 6 shows the detection of a single clinical sample using different combinations of primers and probes;
FIG. 7 shows detection of a reference sample using two sets of primers and probes with the primer of SEQ ID NO:6;
FIG. 8 shows detection of a reference sample using two sets of primers and probes without the primer of SEQ ID NO:6.

### DETAILED DESCRIPTION OF THE EMBODIMENTS

Hereinafter, the present application will be described in detail with reference to specific embodiments and examples. The advantages and various effects of the present application will be more clearly presented therefrom. It should be understood by those skilled in the art that these specific embodiments and examples are intended to illustrate, but not to limit, the present application.

Example 1. Primers and probes used in the present disclosure:
forward primer HIV-ZF1: 5'-CCTGGGAGCTCTCTGGCTA-3' (SEQ ID NO: 1);
reverse primer HIV-ZR1: 5'-AAGCACTCAAGGCAAGCTTTAT-3' (SEQ ID NO: 2);
detection probe HIV-ZP1: 5' AGGCTTAAGCAGTGGGTTCC-3' (SEQ ID NO:3);
forward primer Z-POL-F1: 5'-AAATCACTCTTTGGCARCGAC-3' (SEQ ID NO: 4);
reverse primer Z-POL-R1: 5'-CCCCCTATCATTTTTGGTTTC-3' (SEQ ID NO:5);
forward primer Z-POL-FO: 5'-ATCCCTCTTTGGGACAGACC-3' (SEQ ID NO:6);
detection probe Z-POL-P1: 5'-ACTGTATCATCTGCYCCTGT-3' (SEQ ID NO:7);
internal standard forward primer HPGT31-F: 5'-CAAAGACCGTTGTGTCCAGAAG-3' (SEQ ID NO:8);
internal standard reverse primer HPGT31-R: 5'-CGCATGTTTACACTTGGGTTTC-3' (SEQ ID NO:9); and
internal standard probe HPGT31-P: 5'-CAACGTCCTGTCCACCTTCCTCCTATG-3' (SEQ ID NO: 10).

Fluorescent groups of the detection probes were FAM, and fluorescent groups of the internal standard probe were HEX.

### Example 2. Method for detecting HIV-1

Extraction of HIV-1 RNA nucleic acid: nucleic acid extraction or purification reagent produced by SANSURE BIOTECH INC.

2.1 20µL proteinase K and 500µL lysis buffer were added to an appropriate number of 1.5 mL centrifuge tubes each.

2.2 After centrifugation at 6000 rpm for 5 min, 800 µL of a sample (plasma) to be tested was added to the above centrifuge tube. The tube was capped and the solution was mixed by shaking for 10 sec.

2.3 After mixed by shaking for 10 sec, the tube was allowed to stand for 30 min.

2.4 After short spin, the centrifuge tube was placed in the magnetic separator for 5 min, and then the solution was gently pipetted out without touching the brown substance adsorbed on the tube wall.

2.5 500 µL of nucleic acid washing solution 1 and 200 µl of nucleic acid washing solution 2 were added and mixed by shaking for 5 sec. After short spin, the centrifuge tube was placed in the separator again.

2.6 After standing for about 3 min, the supernatant was divided into two layers. The pipette tip was inserted into the bottom of the centrifuge tube, and the liquid was gently pipetted out entirely from the bottom and discarded. After standing for 1 minute, the residual liquid at the bottom of the tube was pipetted out and discarded completely.

2.7 35 µL of elution solution was added to elute the magnetic beads from the wall to the bottom of the centrifuge tube. After mixed by pipetting for 3 to 4 times and allowed to stand at room temperature for 10 minutes, the centrifuge tube was placed in the separator again for 3 min. The eluted nucleic acid was placed in a new 1.5 mL sterile centrifuge tube.

30 µL of the eluted nucleic acid was pipetted to PCR reaction solution and subjected to PCR amplification for detection.

### Nucleic acid PCR amplification:

3.1 The PCR reaction tubes were put into sample wells of the amplifier with the negative control, positive control, quantitative reference products A~D and the unknown samples set in the corresponding order, and the name of the sample and the concentration of the quantitative reference product were set.

3.2 Fluorescence detection channel selection

3.2.1 ABI instrument and Hongshi SLAN instrument:
1) FAM channel (Reporter: FAM, Quencher: none) was selected to detect HIV-RNA; 2) HEX or VIC channel (Reporter: HEX/VIC, Quencher; none) was selected to detect HIV internal standard; and 3) Reference fluorescence (Reference Dye) ): ROX was selected.

3.2.2 Roche fluorescent PCR instrument:
New Experiment option was selected, and Dual Color Hydrolysis Probe/UPL Probe was selected in the drop-down menu of Detection format on the setup panel. In the drop-down menu Customize: 1) FAM channel was selected to detect HIV-RNA; and 2) VIC/HEX/Yellow channel was selected to detect HIV internal standard. Reaction Volume was set to 60.

3.3 setup of the cycle parameter:

| | Step | Temperature | Time | Number of cycles |
|---|---|---|---|---|
| 1 | Pre-denaturation and enzyme activation | 95 °C | 1 min | 1 |
| 2 | Reverse transcription | 60 °C | 30 min | 1 |
| 3 | cDNA pre-denaturation | 95 °C | 1 min | 1 |
| 4 | Denaturation | 95 °C | 15 sec | |
| | Annealing, extending and fluorescence collecting | 58 °C | 30 sec | 45 |
| 5 | Cooling instrument | 25 °C | 10 sec | 1 |

### 4. Analysis of Results

After the reaction was completed, the results were automatically saved, and the curve for the human immunodeficiency virus and the curve for the corresponding human immunodeficiency virus internal standard were analyzed respectively. According to the image after analysis, Start value, End value and Threshold value of Baseline were adjusted (Start value may be set at 3~15, End value may be set at 5~20, and the amplification curve for the negative control can be adjusted to be horizontal and straight or below the threshold line). After clicking Analyze button for analysis, the quantitative results were recorded in the Plate window.

### Example 3. Detection of clinical samples using the composition of the present application

Clinical samples were used in the experiment for comparison. A total of 500 plasma samples were tested, including 415 males and 85 females, with an age ranging from 10 to 80 years old. Various types of information were obtained by sequencing the samples, including 69 cases of B subtype (including B type and B' type), 176 cases of BC subtype (including CRF07 _BC and CRF08 _BC type) and 140 cases of AE subtype (including CRF01_AE type). The composition in Example 1 and the control reagent were used for detection according to the method described in Example 2. The results are shown in Table 1.

**Table 1. Distribution of results of clinical samples tested with the present reagent**

| Test results (IU/mL) | Number (case) of samples detected with the present reagent | Number (case) of samples detected with the control reagent |
|---|---|---|
| 1E7~1E8 | 8 | 7 |
| 1E6~1E7 | 38 | 38 |
| 1E5~1E6 | 107 | 109 |
| 1E4~1E5 | 110 | 108 |
| 1E3~1E4 | 63 | 63 |
| 250~1E3 | 46 | 44 |
| 20~250 | 83 | 85 |
| <20(negative) | 45 | 46 |

It can be seen from the results in Table 1 that the clinical samples can be accurately detected with the composition of the present application. Limited by the minimum detection limit, the control reagent has a low detection efficiency for samples with a viral load below 25 IU/mL, resulting in one more sample with a viral load below 25 IU/mL detected using the control reagent than that using the present reagent, that is, the number of the detected positive samples using the control reagent was cut down by one . However, using the composition of the present application, samples below 25 IU can be detected. therefore, no false negative occurred.

### Example 4. Sensitivity tests of the composition of the present application

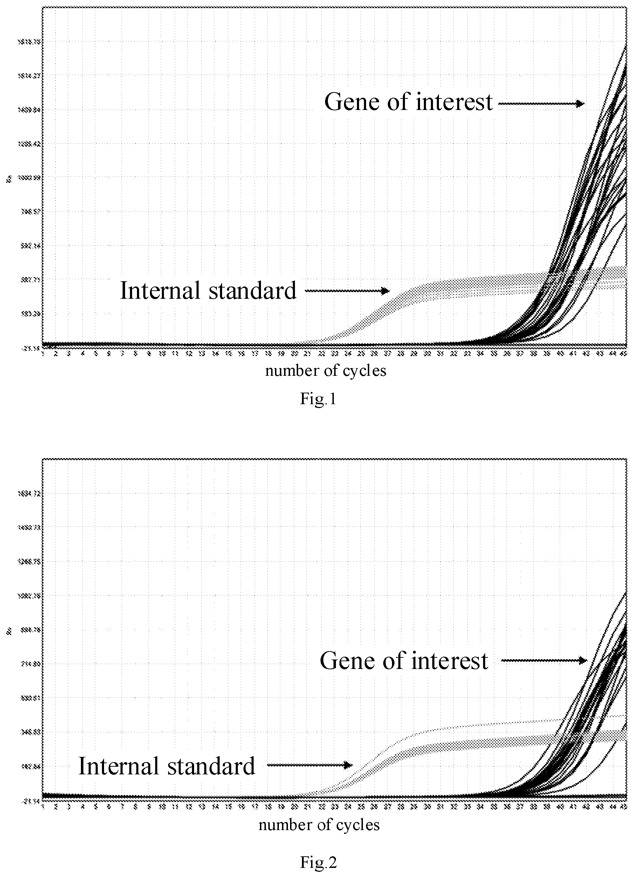

This test was performed by diluting the reference product of known concentration to 25IU/mL and 22IU/mL and using the composition in Example 1 according to the method described in Example 2. The results are shown in FIGS. 1-2. The experimental result shows that a sample of 22 IU/mL can be detected using the composition of the present application.

### Comparative Example 1. Detection of HIV-1 using a comparative composition

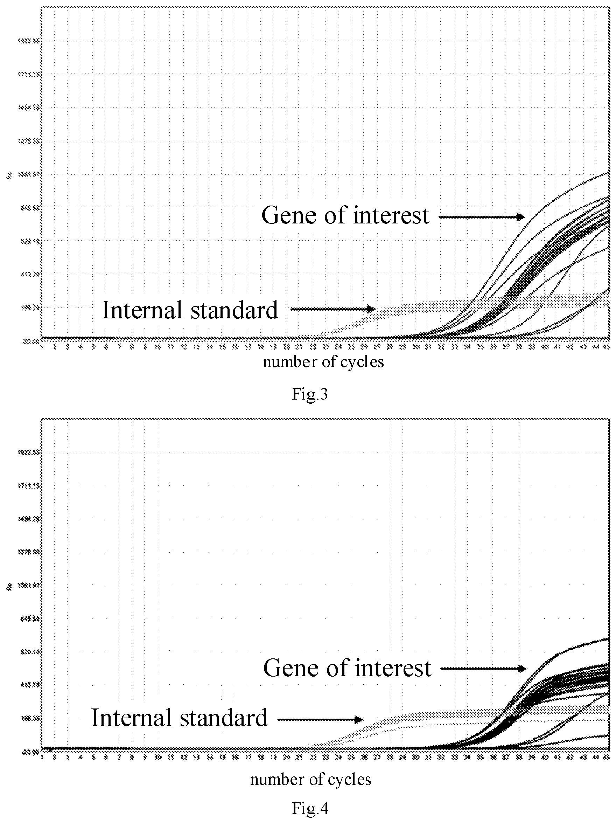

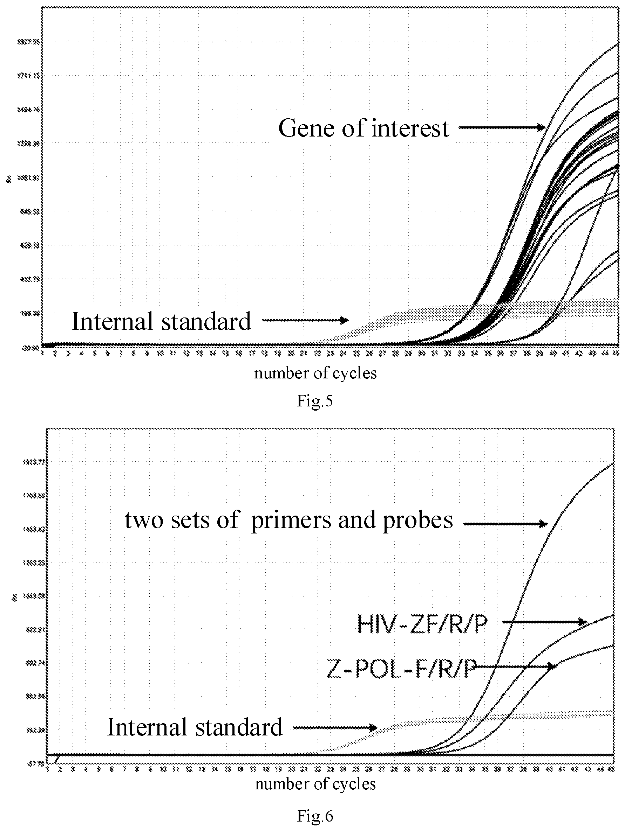

In this comparative experiment, clinical samples were used and 24 cases of plasma were tested. The composition in Example 1 and the control reagent were used for detection according to the method described in Example 2. The results are shown in Figs. 3-6.

From the comparison results, it was found that the detection rate for amplification with single-target primer-probe were consistent with that for amplification with dual-target primer-probe combination. All 24 clinical samples could be detected. However, amplification curve with the single-target primer-probe has a lower fluorescence signal intensity than that with the dual-target primer-probe combination, and the detection curve with the single-target primer-probe has a delayed CT value compared with the dual-target primer-probe combination.

### Comparative Example 2. Comparative detection of HIV- 1 with or without primer of SEQ ID NO:6 added

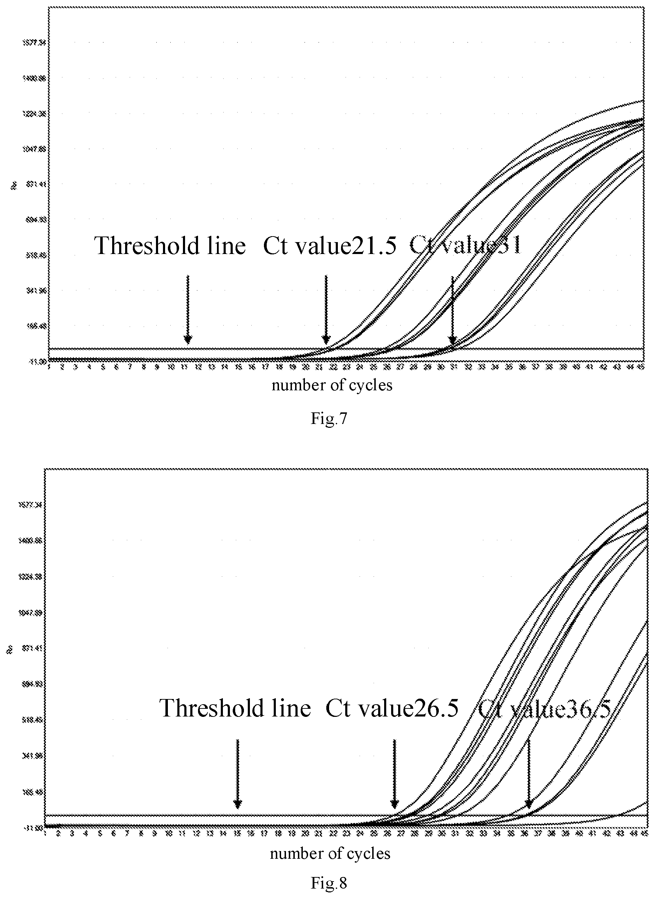

In this scheme, an HIV-1 group O reference sample at a determined concentration was diluted in equal proportion, and then detected using a dual detection system with or without the primer of SEQ ID NO:6 added. The test results are shown in Figures 7-8.

As a result of this scheme, with the primer of SEQ ID NO:6, the HIV-1 group O reference sample was detected with a CT value between 21.5 and 26.5 and a detection rate of 100% (12/12); while without the primer of SEQ ID NO:6 added, the HIV-1 group O reference sample at the same concentration was detected with a CT value between 26.5 and 36.5 and a lower detection rate of 91.7% (11/12). Moreover, addition of the primer of SEQ ID NO:6 resulted in an average reduction of CT value by 5, a better curve, and a higher detection rate for samples at a low concentration, indicating that adding the primer of SEQ ID NO:6 improves the sensitivity of the entire composition for detecting HIV, particularly HIV-1 group O, as compared to the detection without the primer of SEQ ID NO:6.

## Claims

1. A composition for detecting HIV-1 comprising:
a forward primer HIV-ZF1: 5'-CCTGGGAGCTCTCTGGCTA-3' (SEQ ID NO: 1);
a reverse primer HIV-ZR1: 5'-AAGCACTCAAGGCAAGCTTTAT-3' (SEQ ID NO: 2);
a detection probe HIV-ZP1: 5'-AGGCTTAAGCAGTGGGTTCC-3' (SEQ ID NO: 3);
a forward primer Z-POL-F1: 5'-AAATCACTCTTTGGCARCGAC-3' (SEQ ID NO: 4);
a reverse primer Z-POL-R1: 5'-CCCCCTATCATTTTTGGTTTC-3' (SEQ ID NO:5);
a forward primer Z-POL-FO: 5'-ATCCCTCTTTGGGACAGACC-3' (SEQ ID NO:6); and
a detection probe Z-POL-P1: 5'-ACTGTATCATCTGCYCCTGT-3' (SEQ ID NO:7).

2. The composition according to claim 1, further comprising an internal standard forward primer, an internal standard reverse primer, and an internal standard probe, for monitoring.

3. The composition according to claim 1, further comprising an internal standard forward primer as set forth in SEQ ID NO:8, an internal standard reverse primer as set forth in SEQ ID NO:9, and an internal standard probe as set forth in SEQ ID NO: 10.

4. The composition according to claim 3, wherein the detection probes have a fluorescent group FAM, and the internal standard probe has a fluorescent group HEX.

5. Use of the composition of any one of claims 1-4 in preparing a kit for detecting HIV-1.

6. A kit for detecting HIV-1, comprising the composition of any one of claims 1-4 or comprising the nucleic acid components of any one of claims 1-4 wherein said components are present in separate packages.

7. The kit according to claim 6, further comprising a nucleic acid release system and a nucleic acid amplification system.

8. The kit according to claim 6 further comprising a standard product.

9. A method for detecting HIV-1 for non-diagnostic purposes, comprising:
1) extracting or releasing nucleic acids from a sample to be tested;
2) subjecting the nucleic acids obtained in 1) to fluorescence quantitative PCR using the composition of any one of claims 1-4; and
3) obtaining a result and analyzing the result.

## Patentansprüche

1. Zusammensetzung zum Nachweis von HIV-1, umfassend:
einen Vorwärtsprimer HIV-ZF1: 5'-CCTGGGAGCTCTCTGGCTA-3' (SEQ ID NO: 1);
einen Rückwärtsprimer HIV-ZR1: 5'-AAGCACTCAAGGCAAGCTTTAT-3' (SEQ ID NO: 2);
eine Detektionssonde HIV-ZP1: 5'-AGGCTTAAGCAGTGGGTTCC-3' (SEQ ID NO:3);
einen Vorwärtsprimer Z-POL-F1: 5'-AAATCACTCTTTGGCARCGAC-3' (SEQ ID NO:4);
einen Rückwärtsprimer Z-POL-R1: 5'-CCCCCTATCATTTTTGGTTTC-3' (SEQ ID NO:5);
ein Vorwärtsprimer Z-POL-FO: 5'-ATCCCTCTTTGGGACAGACC-3' (SEQ ID NO:6); und
eine Detektionssonde Z-POL-P1: 5'-ACTGTATCATCTGCYCCTGT-3' (SEQ ID NO:7).

2. Zusammensetzung gemäß Anspruch 1, ferner umfassend einen internen Standard-Vorwärtsprimer, einen internen Standard-Rückwärtsprimer und eine interne Standard-Sonde zur Überwachung.

3. Zusammensetzung gemäß Anspruch 1, ferner umfassend einen internen Standard-Vorwärtsprimer wie in SEQ ID NO:8 aufgeführt, einen internen Standard-Rückwärtsprimer wie in SEQ ID NO:9 aufgeführt und eine interne Standard-Sonde wie in SEQ ID NO:10 aufgeführt.

4. Zusammensetzung gemäß Anspruch 3, wobei die Detektionssonden eine Fluoreszenzgruppe FAM aufweisen und die interne Standard-Sonde eine Fluoreszenzgruppe HEX aufweist.

5. Verwendung der Zusammensetzung gemäß einem der Ansprüche 1 bis 4 zur Herstellung eines Kits zum Nachweis von HIV-1.

6. Kit zum Nachweis von HIV-1, umfassend die Zusammensetzung gemäß einem der Ansprüche 1 bis 4 oder umfassend die Nukleinsäurekomponenten gemäß einem der Ansprüche 1 bis 4, wobei die Komponenten in separaten Verpackungen vorliegen.

7. Kit gemäß Anspruch 6, ferner umfassend ein Nukleinsäurefreisetzungssystem und ein Nukleinsäureamplifikationssystem.

8. Kit gemäß Anspruch 6, ferner umfassend ein Standardprodukt.

9. Verfahren zum Nachweis von HIV-1 für nicht-diagnostische Zwecke, umfassend:
1) Extrahieren oder Freisetzen von Nukleinsäuren aus einer zu testenden Probe;
2) Unterziehen der in 1) erhaltenen Nukleinsäuren einer quantitativen Fluoreszenz-PCR unter Verwendung der Zusammensetzung gemäß einem der Ansprüche 1 bis 4; und
3) Erhalten eines Ergebnisses und Analysieren des Ergebnisses.

## Revendications

1. Une composition pour détecter le VIH-1 comprenant :
une amorce directe HIV-ZF1 : 5'-CCTGGGAGCTCTCTGGCTA-3' (SEQ ID NO : 1) ;
une amorce inverse HIV-ZR1 : 5'-AAGCACTCAAGGCAAGCTTTAT-3' (SEQ ID NO : 2) ;
une sonde de détection HIV-ZP1 : 5'-AGGCTTAAGCAGTGGGTTCC-3' (SEQ ID NO : 3) ;
une amorce directe Z-POL-F1 : 5'-AAATCACTCTTTGGCARCGAC-3' (SEQ ID NO : 4) ;
une amorce inverse Z-POL-R1 : 5'-CCCCCTATCATTTTTGGTTTC-3' (SEQ ID NO : 5) ;
une amorce directe Z-POL-FO : 5'-ATCCCTCTTTGGGACAGACC-3' (SEQ ID NO : 6) ; et
une sonde de détection Z-POL-P1 : 5'-ACTGTATCATCTGCYCCTGT-3' (SEQ ID NO:7).

2. Composition selon la revendication 1, comprenant en outre une amorce directe standard interne, une amorce inverse standard interne et une sonde standard interne, à des fins de surveillance.

3. Composition selon la revendication 1, comprenant en outre une amorce interne standard en sens direct telle que décrite dans SEQ ID NO:8, une amorce interne standard en sens inverse telle que décrite dans SEQ ID NO:9, et une sonde interne standard telle que décrite dans SEQ ID NO:10.

4. Composition selon la revendication 3, dans laquelle les sondes de détection ont un groupe fluorescent FAM et la sonde standard interne a un groupe fluorescent HEX.

5. Utilisation de la composition selon l'une quelconque des revendications 1 à 4 dans la préparation d'un kit pour la détection du VIH-1.

6. Kit pour détecter le VIH-1, comprenant la composition selon l'une quelconque des revendications 1 à 4 ou comprenant les composants d'acide nucléique selon l'une quelconque des revendications 1 à 4, dans lequel lesdits composants sont présents dans des emballages séparés.

7. Kit selon la revendication 6, comprenant en outre un système de libération d'acide nucléique et un système d'amplification d'acide nucléique.

8. Kit selon la revendication 6, comprenant en outre un produit standard.

9. Procédé de détection du VIH-1 à des fins non diagnostiques, comprenant :
1) l'extraction ou la libération d'acides nucléiques à partir d'un échantillon à tester ;
2) soumettre les acides nucléiques obtenus en 1) à une PCR quantitative par fluorescence en utilisant la composition de l'une quelconque des revendications 1 à 4; et
3) l'obtention d'un résultat et l'analyse du résultat.
